# EUROPEAN PATENT SPECIFICATION

(11) **EP 0 544 950 B1**
(45) Date of publication and mention of the grant of the patent: **15.01.1997**
(21) Application number: 91311153.0
(22) Date of filing: 29.11.1991
(51) Int. Cl.: A61K 33/00

(54) **Chlordioxide containing agent against fishparasites**
Chlordioxid enthaltendes Mittel gegen Fischparasiten
Agent contenant du dioxyde de chlore contre les parasites des poissons

(43) Date of publication of application: 09.06.1993
(73) Proprietor: JAPAN PET DRUGS CO., LTD., Katsushika-Ku, Tokyo (JP)
(72) Inventor: Yoshida, Nobuyuki, Katsushika-ku, Tokyo (JP)
(74) Representative: Baillie, Iain Cameron

(56) References cited:
- US-A- 4 946 690
- PATENT ABSTRACTS OF JAPAN, vol. 12, no. 491 (C-554)[3338], 21st December 1988; & JP-A-63 201 129

## Description

This invention relates to the use of stabilized chlorine dioxide in a method of preparing a medium to cure red-sore disease of aquarium fish wherein the concentration in said medium of chlorine dioxide is at least 20 ppm.

Red-sore disease is a parasitosis which is caused by parasitism of Epistylis longicorpora on the body surface or scales of fish.

For curing this disease, there has been commonly employed treatment with medicated water - placing the diseased fish in water containing a certain dye such as malachite green or methylene blue or other medicinal substance such as formaline, common salt, etc. When this disease is complicated by a bacterially caused disease, an antibacterial agent such as furanoid is used with said chemicals.

Treatment of red-sore disease by use of these drugs has the problems such as mentioned below.
1) Dye drugs tint water in the water tank where aquarium fish are kept, making it difficult to enjoy the appearance of aquarium fish in the water tank. Also, dye drugs are relatively unstable in water since the molecules thereof are bound to oxygen in water.
2) Formalin is poisonous to fish, and excess application of this agent may cause death of fish. Therefore, formalin is unsuited for a long-time medicated water cure.
3) Treatment in water containing common salt is employed for curing of various diseases of aquarium fish, but no remarkable curative effect can be expected. (It has very little effect for treatment of fish in the medium-stage or advanced stage of a disease.) Also, among the tropical fish native to the Amazon in South America, there are many species which are sensitive to common salt (such as small-sized characin and American cichlid), and treatment in salted water is unsuited for these fish.

Thus, it has been desired to develop a curative drug for the diseases of aquarium fish which drug does not cause tinting of water in a water tank and is relatively stable in water and safe to aquarium fish (including tropical fish).

Epistylis longicorpora which cause red-sore disease is a protozoan belonging to stalked Ciliata.

In the life cycle of this protozoan, the fully developed body shrinks to a sphere on a fish body and the cilia grow annularly near the rear portion of the body. Then the body is flattened and develops the cilia around it. Finally, it leaves the stalk and freely swims in water, which allows dissemination of this protozoan. It becomes a parasite on a new host and grows to a stalked body.

A peculiarity of lesion by red-sore disease is destruction of tissue which occurs when Epistylis stays parasitic on the fish body. Since the destroyed tissues are infected with ― pathogenic bacteria, the diseased fish tend to be concurrently affected with a bacterial disease.

Therefore, for cure of red-sore disease, unlike the case of treatment of other parasitoses, there is a need to get rid of Epistylis longicorpora itself which is parasitic on the fish body. In this case, it may be required to additionally give an antibacterial drug for preventing secondary infection with the bacteria.

As a result of extensive studies for discovering an effective curative method for red-sore disease, the present inventors found that stabilized chlorine dioxide which is effective for cure of ichtyophthiriasis of colored carp or goldfish (see U.S. Patent No. 4,946,690) is also effective for treatment of red-sore disease. The present invention was attained on the basis of this finding.

Thus the present invention is directed to the use of stabilized chlorine dioxide in a method of preparing a medium to cure red-sore disease of aquarium fish wherein the concentration in said medium of chlorine dioxide is at least 20 ppm.

Stabilized chlorine dioxide used for said purpose in the present invention is applied to a place where aquarium fish live, for example, a water tank in which the aquarium fish such as colored carp, goldfish and tropical fish are kept. Stabilized chlorine dioxide is added in such an amount that the concentration thereof in water will become 20 ppm or above, preferably 20 to 30 ppm.

A large difference between the present invention and the method for exterminating Ichthyophthirius multiliis is that in the case of Ichthyophthirius multiliis, stabilized chlorine dioxide takes effect only against the telotroch in the life cycle of this ciliate, whereas in the case of Epistylis, since in the life cycle thereof there is no period in which the adult ciliate swims away from the host fish and finds a new fish body that can serve as a host for the telotroch, it is necessary to get rid of matured Epistylis. It was surprisingly found, however, that it is possible with stabilized chlorine dioxide to exterminate Epistylis although a slightly higher concentration is necessary than required for killing Ichthyophthirius multiliis in the stage of telotroch, and this finding was materialized as the present invention.

The effect of the present invention is described below with reference to the test examples.

### Test of curative effect on red-sore disease

### Test method

### 1. Epistylis exterminating concentration of stabilized chlorine dioxide (in vitro)

The regions infected with the Epistylis-affected fish (goldfish having parasite colonies on the caudal fin) which had been injected by the disease by themselves were cut off, and the cut-off infected regions (parasitic colonies of Epistylis) were put into beakers containing 30 ml of aqueous solutions with ClO₂ concentrations of 0 ppm, 5 ppm, 10 ppm, 20 ppm and 30 ppm, respectively. After being kept in said beakers at 26°C for the periods of 12 hours, 24 hours 36 hours and 48 hours, said regions were taken out and the state of Epitylis in said regions was observed.

### 2. Curing test of red-sore disease by stabilized chlorine dioxide

There were prepared four water tanks each containing 55 litres of water, and in each water tank were placed 15 infected fish (same in stage of parasitism). ClO₂ was put into the water tanks at concentrations of 0 ppm, 10 ppm, 20 ppm and 30 ppm, respectively. The change of condition of the infected fish was observed for five days and the curative effect of ClO₂ in each test section was examined.

### Results

The in vitro Epistylis exterminating concentrations of ClO₂ are shown in Table 1.

At the ClO₂ concentrations of 20 ppm and 30 ppm, almost all of the parasites (Epistylis) were dead in 24 hours. At the ClO₂ concentrations of 5 ppm and 10 ppm, the parasites still showed active movement 24 hours after start of the test, but their movement became sluggish in 48 hours at the ClO₂ concentration of 5 ppm and in 36 hours at the ClO₂ concentration of 10 ppm. In the control section (ClO₂ concentration = 0 ppm), the parasites kept moving actively throughout the test period.

From the above results, it was confirmed that ClO₂ was effective for exterminating Epitylis.

It is supposed that a ClO₂ concentration of 20 ppm will be effective for the actual curing treatment of Epistylis-affected fish. This level of ClO₂ concentration does the aquarium fish no harm.

The results of the actual curing tests are shown in Table 2. As seen from the table, the cure rate in the test sections with ClO₂ concentrations of 20 ppm and 30 ppm was 86.7%. In contrast, in the test section with 0 ppm of ClO₂, 11 fish died and the condition of the disease was worsened in 2 fish. Most of these infected fish were congested ruddily not merely at the infected region but all over the body and had a complication of a typical bacterially caused disease. From these results, it was found that treatment in water containing ClO₂ at a concentration of 20 to 30 ppm is effective for cure of red-sore disease of aquarium fish.

**Table 1**

| Condition of epistylis at various concentrations of ClO₂ | | | | |
|---|---|---|---|---|
| | Condition of Epistylis | | | |
| | After 12 hrs. | After 24 hrs. | After 36 hrs. | After 48 hrs. |
| 0 ppm | ○ | ○ | ○ | ○ |
| 5 ppm | ○ | ○ | ○ | △ |
| 10 ppm | ○ | ○ | △ | △ |
| 20 ppm | ○ | X | X | X |
| 30 ppm | ○ | X | X | X |
| ○ : The parasites kept moving actively. △ : Movement of the parasites became sluggish. (More than half of the parasites in the colony died.) X : More than 90% of the parasites in the colony died. | | | | |

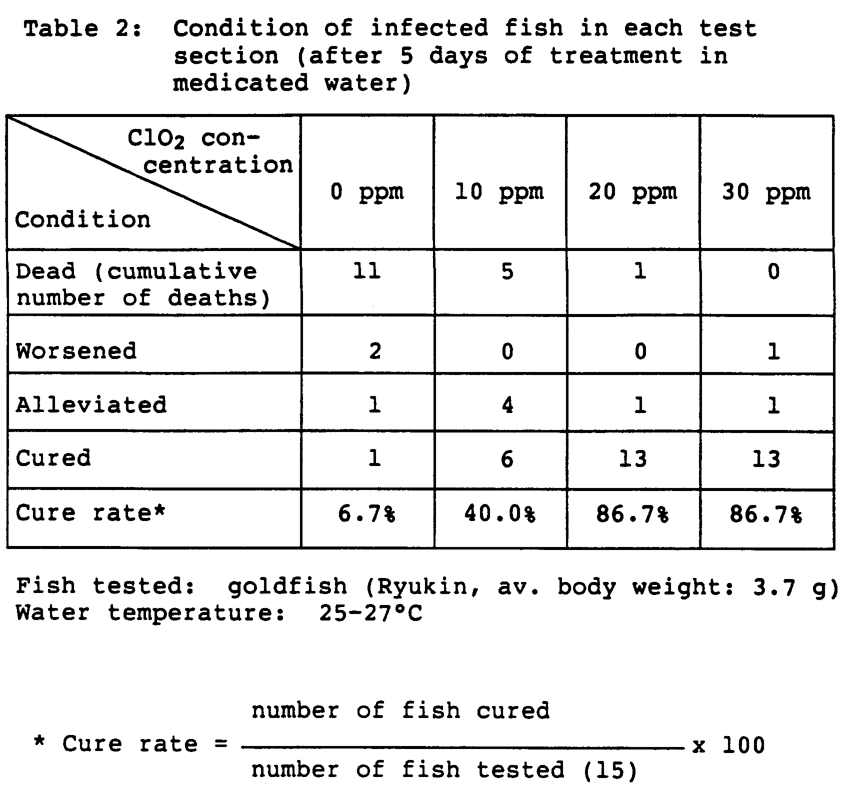

### Test of ClO₂ stability in water

The stability of ClO₂ in water in a water tank commonly employed for keeping aquarium fish was examined by changing the tank environment.

The condition of the water tank was changed (by aerating (○) or not aerating (X) the tank and illuminating (○) or not illuminating (X) the tank) and the influence on the agent was checked over a period of 20 days. There was noted little difference (in ClO₂ concentration) after the test as seen from Table 3.

**Table 3**

| Water tank | Aeration | Illumination | Initial | After 7 days | After 14 days | After 20 days |
|---|---|---|---|---|---|---|
| 1 | X | X | 5.50 ppm | 5.33 ppm | 5.39 ppm | 5.27 ppm |
| 2 | ○ | ○ | 5.38 ppm | 5.30 ppm | 5.18 ppm | 5.15 ppm |

Test was further made on ClO₂ stability by applying both aeration and illumination to the water tank and placing (○) or not placing (X) 10 goldfish in the tank.

As a result, as seen from Table 4, the ClO₂ concentration remained above 5 ppm throughout the test period of 20 days even when 10 goldfish were placed in the water tank, indicating high stability of ClO₂ in water regardless of said changes in environment of the water tank.

**Table 4**

| Water tank | Goldfish | ClO₂ | Initial | After 7 days | After 14 days | After 20 days |
|---|---|---|---|---|---|---|
| 1 | ○ | ○ | 5.41 ppm | 5.53 ppm | 5.44 ppm | 5.32 ppm |
| | | | pH 7.94 | pH 8.02 | pH 8.05 | pH 8.06 |
| 2 | ○ | X | pH 7.93 | pH 8.08 | pH 7.93 | pH 8.07 |
| 3 | X | ○ | 5.73 ppm | 5.62 ppm | 5.67 ppm | 5.89 ppm |
| | | | pH 8.06 | pH 8.10 | pH 8.03 | pH 8.11 |

As apparent from the above tests, stabilized chlorine dioxide is useful as a curative agent for red-sore disease and meets all of aid requirements in practical application.

The utility of stabilized chlorine dioxide in use in water for treatment of fish suffering from red-sore disease is itemized below.

### 1) Stabilized chlorine dioxide does not tint water.

Since stabilized chlorine dioxide is a colorless transparent liquid, it does not tint water in a water tank. Therefore, it gives no adverse effect on enjoyment of aquarium fish.

### 2) Stabilized chlorine dioxide is stable in water.

The useful period of dye drugs in water is usually about 5 days, but there are the cases where it is difficult to cure fish of the disease within this period. Stabilized chlorine dioxide remains stable in water even after the lapse of 20 days from its addition into water. Therefore, it enables treatment of infected fish by placing them only once in medicated water in a tank.

### 3) Stabilized chlorine dioxide can exterminate stalked Epistylis parasitic on fish.

Regarding effect on other parasites (e.g. Ichthyophthirius multiliis), it has only been known that stabilized chlorine dioxide has an exterminating action against telotroch alone, but it was found for the first time by the present inventors that this compound also has an exterminating action on adult Epistylis.

### 4) Stabilized chlorine dioxide prevents secondary infection of bacteria.

As mentioned before, red-sore disease tends to cause secondary bacterial infection, so that for cure thereof, a drug efficacious against this disease has been given along with an antibacterial agent. However, it is possible with stabilized chlorine dioxide, at a concentration (20 ppm) effective for cure of red-sore disease, to destroy the germs existing in water. Thus, it is possible to prevent secondary bacterial infection by application of stabilized chlorine dioxide alone. It was disclosed for the first time by the present inventors that stabilized chlorine dioxide can exterminate Epistylis and is also efficacious against the bacterial infectious diseases which tend to occur concomitantly with red-sore disease.

As appreciated from the foregoing, stabilized chlorine dioxide is an agent that can eliminate all of the problems of the conventional curative drugs for red-sore disease.

## Claims

1. Use of stabilized chlorine dioxide in a method of preparing a medium to cure red-sore disease of aquarium fish wherein the concentration in said medium of chlorine dioxide is at least 20 ppm.

2. A use according to claim 1, wherein the aquarium fish are coloured carp.

3. A use according to any preceding claim, wherein the concentration of said stabilized chlorine dioxide is 20 to 30 ppm.

## Patentansprüche

1. Verwendung von stabilisiertem Chlordioxid in einem Verfahren zur Herstellung eines Mittels zur Behandlung der "Red-Sore"-Erkrankung von Zierfischen, wobei die Konzentration von Chlordioxid in dem Mittel mindestens 20 ppm beträgt.

2. Verwendung nach Anspruch 1, bei der die Zierfische Buntkarpfen sind.

3. Verwendung nach einem der vorhergehenden Ansprüche, bei der die Konzentration des stabilisierten Chlordioxids 20 bis 30 ppm beträgt.

## Revendications

1. Utilisation de dioxyde de chlore stabilisé dans un procédé de préparation d'un milieu pour guérir la maladie due à *Epistylis longicorpora* chez les poissons d'aquarium, où la concentration de dioxyde de chlore dans ledit milieu est d'au moins 20 ppm.

2. Utilisation selon la revendication 1, où les poissons d'aquarium sont des cyprins.

3. Utilisation selon l'une ou l'autre des revendications 1 et 2, où la concentration dudit dioxyde de chlore stabilisé est de 20 à 30 ppm.
